# EUROPEAN PATENT SPECIFICATION

(11) **EP 4 176 265 B1**
(45) Date of publication and mention of the grant of the patent: **13.08.2025**
(21) Application number: 21743390.3
(22) Date of filing: 02.07.2021
(51) Int. Cl.: G01N 33/569, G01N 33/543

(54) **KITS AND METHODS FOR THE ENRICHMENT AND DETECTION OF RNA VIRUSES OF THE CORONAVIRIDAE FAMILY**
KITS UND VERFAHREN ZUR ANREICHERUNG UND ZUM NACHWEIS VON RNA-VIREN DER FAMILIE CORONAVIRIDAE
KITS ET MÉTHODES D'ENRICHISSEMENT ET DE DÉTECTION DE VIRUS À ARN DE LA FAMILLE DES CORONAVIRIDAE

(30) Priority: 03.07.2020 DE 102020117636
(43) Date of publication of application: 10.05.2023
(73) Proprietor: Johannes Gutenberg-Universität Mainz, 55122 Mainz (DE)
(72) Inventor: HÜLSMANN, Bastian, 55116 Mainz (DE)
(74) Representative: Patentanwälte Dr. Keller, Schwertfeger Partnerschaft mbB
(86) International application number: PCT/EP2021/068395
(87) International publication number: WO 2022/003180

(56) References cited:
- CN-A- 102 221 611
- DE-A1- 102016 121 455
- US-B2- 9 632 078
- HUANG LIPING ET AL: "One-Step Rapid Quantification of SARS-CoV-2 Virus Particles via Low-Cost Nanoplasmonic Sensors in Generic Microplate Reader and Point-of-Care Device", BIORXIV, 12 June 2020 (2020-06-12), XP055847799, Retrieved from the Internet <URL:https://www.biorxiv.org/content/10.1101/2020.06.09.142372v2.full.pdf> [retrieved on 20211005], DOI: 10.1101/2020.06.09.142372
- MAYOR H D ET AL: "Studies on the acridine orange staining of two purified RNA viruses: Poliovirus and tobacco mosaic virus", VIROLOGY, ELSEVIER, AMSTERDAM, NL, vol. 14, no. 1, 1 May 1961 (1961-05-01), pages 74 - 82, XP023052960, ISSN: 0042-6822, [retrieved on 19610501], DOI: 10.1016/0042-6822(61)90134-9
- ROBB NICOLE C ET AL: "Rapid functionalisation and detection of viruses via a novel Ca2+-mediated virus-DNA interaction", SCIENTIFIC REPORTS,, vol. 9, no. l, 7 November 2019 (2019-11-07), pages 1 - 14, XP002803492, DOI: 10.1038/S41598-019-52759-5

## Description

### TECHNICAL FIELD

The present invention relates to kits and methods for the detection of SARS-CoV-2 RNA viruses of the *Coronaviridae* family.

### BACKGROUND ART

Coronaviruses belong to the family *Coronaviridae* and mainly cause respiratory tract infections of varying severity. *Coronaviridae* is a family of enveloped, positive-strand RNA viruses which infect amphibians, birds, and mammals. The group includes the subfamilies *Letovirinae* and *Orthocoronavirinae.* Seven human coronaviruses (HCoVs) have been so far identified, namely HCoV-229E, HCoV-OC43, HCoV-NL63, HCoV-HKU1, severe acute respiratory syndrome coronavirus (SARS-CoV), Middle East respiratory syndrome coronavirus (MERS-CoV) and the novel coronavirus (2019-nCoV, also known as SARS-CoV-2). They are responsible for one third of all colds worldwide. SARS-CoV appeared in the human population in 2002, MERS-CoV appeared in Saudi Arabia in 2012, and SARS-CoV-2, which appeared for the first time in Wuhan, China, in late 2019. The novel coronavirus SARS-CoV-2 causes the lung disease COVID-19. Novel human coronaviruses are of zoonotic origin, meaning they pass from animals to humans. Bats are considered natural reservoirs for SARS-CoVs, and the dromedary for MERS-CoV. Common signs of infection are respiratory symptoms, fever, cough, shortness of breath. In severe cases, the infection can also lead to pneumonia or kidney failure.

Coronaviruses of the subfamily Orthocoronavirinae are divided into α-, β-, y- and δ-coronaviruses, and consist of a helical nucleocapsid enveloped by a lipid membrane in which the structural proteins E (envelope protein), S (spike glycoprotein) and M (membrane protein) are embedded. The S protein is responsible for binding to cellular receptors and for the subsequent entry of the virus into the target cells. This can only take place if the S protein is functionally cleaved by cellular proteases. Due to its function, this protein is the target for neutralising antibodies.

Receptor binding is critical to initiate viral infection. Humane coronaviruses have been shown to use either cellular proteins or carbohydrates displayed on the plasma membrane as receptors. Interestingly, all known protein receptors for human coronaviruses are cell surface peptidase, such as aminopeptidase N (APN) for HCoV-229E, dipeptidyl peptidase 4 (DPP4) for MERS-CoV, and angiotensin converting enzyme 2 (ACE2) for HCoV-NL63, SARS-CoV and SARS-CoV-2.

Coronaviruses are highly variable RNA viruses that can develop new variants very quickly with the help of nucleotide exchange and recombination. These new variants are the basis for zoonotic exchange between species. The RNA genome of coronaviruses is between 26 and 32 kilobases in size. Two-thirds of the genome consists of two overlapping reading frames that encode the complex viral replicase-transcriptase enzyme complex and, after cell entry, translate the virus directly from the viral genome as a precursor polyprotein. After autocatalytic cleavage, the mixed-structure protein subunits that form the functional transcriptase-polymerase complex are produced. The tasks of which are the transcription of viral subgenomic mRNA and the replication of viral genomic RNA. The last third of the genome codes for the viral structural proteins, each of which is translated with the help of the corresponding subgenomic mRNA.

In vitro detection of RNA from β-coronaviruses has so far only been carried out reliably with the help of the real-time polymerase chain reaction (real-time PCR, RT-PCR) by means of panel diagnostics, which can detect different pathogens simultaneously. The biological samples are obtained via a swab from the throat or sputum, for example. The test has a systemic control based on human β-actin genes.

The current RT-PCR test methods detect different gene segments of the coronavirus. Due to the high mutation rates of RNA viruses, two gene segments are often analysed to exclude false-negative results.

The COVID-19 pandemic has shown that existing diagnostics quickly reach their limits, firstly because the number of samples exceeds the testing capacities of the laboratories, and secondly because the detection of RNA-based viruses via RT-PCR is very material- and time-consuming and requires specialised personnel. There are usually several days between the time the sample is taken and the test result, a period during which the course of the disease in the tested persons can deteriorate drastically or during which symptom-free but infectious test persons can infect other people. Furthermore, there are often not enough PCR reagents available, and many hospitals are not equipped with a PCR infrastructure for testing. For this reason, attempts are being made to develop alternative testing methods. Nucleic acid tests using isothermal amplification are currently under development for SARS-CoV-2. Isothermal amplification is performed at a specific temperature and involves a LAMP (loop-mediated isothermal amplification) polymerase reaction, i.e. a loop-mediated isothermal amplification using a DNA polymerase.

In the meantime, SARS-CoV-2 has also been detected in the stool of patients and in a preliminary study, virus particles could even be amplified from samples of sewage (G. Medema et al., 2020, Presence of SARS-Coronavirus-2 in sewage, https://doi.org/10.1101/2020.03.29.20045880).

Due to the limited availability of the kits and the false negative rate of RT-PCR, alternative methods for the diagnosis of coronavirus-related diseases, especially COVID-19, have been developed. These include, for example, computer tomography as well as nucleic acid or protein testing, for example based on the detection of viral protein antigens by antibodies. Serological tests are being developed as well. Point-of-care tests are used to diagnose patients without having to send samples to a centralised site. For this type of tests, nanoparticle-antibody conjugates are used to detect SARS-CoV-2 in a patient sample (e.g. blood or urine). The complex is run through a membrane strip in a commercial lateral flow assay so that the complex is immobilised by the antibodies and becomes visible as a red or blue line. In addition, there are also microfluidic devices equipped with micrometer channels and reaction chambers in a chip.

Amplification methods for the detection of coronaviruses are described, for example, in EP 1 493 822 A1, and a test kit for the immunological diagnosis of a SARS virus infection using a nucleocapsid protein N is part of EP 2 023 142 A1. A similar method is described in Shang Bo, et. al. Characterization and application of monoclonal antibodies against N protein of SARS-coronavirus, Biochemical and Biophysical Research Communications 336 (2005) 110-117. Most assays use monoclonal antibodies and are ELISA-bases such as described in US 2009/0280507A1, CN 102221611A, CN111303254 A. DE 10 2009 032 502 describes a method for the detection of an antigen that comprises the use of antigen-coated beads.

One disadvantage of the existing methods is that they are complex and, in some cases, very time-consuming and material-intensive to perform, which makes it necessary to employ skilled personnel to take the samples and carry out the procedure. Incorrectly taken samples are often the main cause of a false-negative test result. Furthermore, with the known methods, differential diagnostics can only be carried out with considerable effort, for example to determine whether a patient is suffering from an influenza disease or a certain type of coronavirus disease.

Immunological detection methods for the virus are usually not sensitive enough to reliably detect virus present. Another disadvantage is that the reagents for this type of test are limited and bottlenecks must always be expected due to the global application during a pandemic.

Furthermore, special laboratory equipment is needed to perform the procedures, which is not available everywhere. Many of the tests are designed to detect the viral genome, which, however, says nothing yet about the infectivity of the virus particles. This means that testing methods generally do not detect infectious virus, as viral genes are usually detectable for longer than there is an actual risk of infection. In order to prove infectivity, virus isolation in a cell culture must be performed

The closest prior art, DE 10 2016 121 455 A1, describes a method for the detection of virus particles using chaser molecules that are immobilised on a substrate and the detection of immobilised virus particles by fluorescence microscopy. However, the method relies on antibodies as chaser molecule.

### DISCLOSURE OF INVENTION

Against this background, it is the object of the present invention to provide an alternative kit and detection method that is highly sensitive and thus suitable for the detection of SARS-CoV-2 RNA viruses of the *Coronaviridae* family. It is furthermore the object of the present invention to provide a method for the detection of coronaviruses and that is suitable to pool samples in a high-throughput process.

These objects are solved by a method as claimed in claim 1. Preferred embodiments can be found in the sub-claims.

The invention relates to a method for the detection of SARS-CoV-2 RNA viruses of the family *Coronoaviridae,* in particular mammalian coronaviruses of the family *Coronaviridae,* comprising: coupling a binding agent selected from the group consisting of an angiotensin-converting enzyme 2 (ACE2), an ACE2 construct, an ACE2 fusion protein, or a modified or mutant ACE2 polypeptide or fusion protein that is capable of binding to a virus component to beads, incubating the beads with the thereon coupled binding agent with a SARS-CoV-2 virus-containing sample, staining the viruses immobilised on the beads with a staining agent for viral RNA, detecting stained virus particles via a physical, chemical or biological detection means.

The beads can be any material that is suitable for coupling the binding agent. The coupling can be specific or non-specific. A covalent coupling of the binding agent to the beads is preferred. For this purpose, the generally known coupling methods, for example carboxylate bonding, can be used. Preferred beads are selected from coated or labelled beads, magnetic beads, fluorescence-labelled beads or Luminex^{™} beads. Alternative carrier materials are polymethyl methacrylate (PMMA) microparticles, polyethylene (PE) microparticles, polypropylene (PP) microparticles, polystyrene (PS) microparticles, carboxylated or animated latex particles, polydimethylsiloxane (PDMS) microparticles, cellulose acetate microparticles, cyclic olefin copolymer (COC) microparticles, protein A/G particles, agarose microparticles, Sepharose microparticles, magnetic microparticles, a hydrogel, a sol-gel, a porous polymer monolith, a porous silicone or a membrane.

In a preferred embodiment the binding agent is biotinylated ACE2 and the beads are streptavidin agarose beads or streptavidin-PMMA.

The beads, can be labelled with one or more staining agents or staining agent mixtures. In a preferred embodiment, the beads are provided with a coating. For the coupling of the binding agent to the beads methods known in the art can be applied, for example covalent coupling. The surface of the microparticles can be modified so that a directional coupling of the biomolecules on the surface is possible, which can further increase the analytical sensitivity. This can be done, for example, by means of spacers, tags, markers or other modifications. Protein biochips that are suitable for coupling the binding agent can also be used as a carrier material. Also filters, mat of fibres, sintered plates or membranes can be used as carrier material. For example, PVDF, nitrocellulose, nylon, polycarbonates or Al2O3 anodisc filters can be utilized.

In a further step, the beads with the thereon coupled binding agent is incubated with a SARS-CoV-2 virus-containing sample. The sample can be a sample from single patient or a pooled labelled sample from a variety of patients. The beads with the thereon immobilised viruses are subsequently stained with a suitable staining agent for viral RNA. Finally, detection of the stained virus particles is performed using a physical, chemical or biological detection means. Physical, chemical or biological detection means according to the present invention comprise physical, chemical or biological detection agents or physical instruments such as a microscope.

The biological detection means include, but are not limited to, enzymatic detection means or antibody-coupled detection means such as secondary antibodies or antibody markers. An example of an enzymatic detection means is the detection of bound viruses using a specific binding agent (e.g. anti-spike antibody) to which an enzyme is coupled, enabling sensitive detection of the virus via a colour change. In a preferred embodiment, the detection is carried out via secondary binding agents, such as antibodies or conjugates that are bound to markers, or staining agents that emit a detectable signal. The associated colour reactions or light emissions allow the detection of the binding agents coupled to the beads, e.g. the detection of primary antibodies that specifically recognise and bind to a virus component. The markers or conjugates consist, for example, of enzymes (preferably peroxidase, alkaline phosphatase), fluorescent markers (e.g. FITC, Alexa-Fluor, Qdot) or biotin. An evaluation in the sense of the present invention is preferably carried out by colorimetry, e.g. via an analysis of the colour change.

The method according to the invention has the advantage that it is easy to perform and robust. At the same time the method exhibits a high sensitivity. These features allow for rapid tests with a high throughput, and hence the scanning for a wide variety of samples. Depending on the respective application and the desired scale, microtiter plates with 8-12 wells, 96 wells, 384 wells or more can be used. The use of a silicon wafer, a chip, a microarray slide or a matrix is also possible. Similarly, bead-based multiplex assays can be used. The analysis and evaluation of the multiplex assays can be carried out, for example, with a Luminex^{™} analysis system, via a FACS analysis device or microfluidic systems (e.g. "lab-on-a-chip" systems). However, for a quick and cost-effective analysis, the staining agent is preferably detectable with the naked eye or by the help of an optical lens, such as the integrated camera of a smartphone. For a more accurate analysis, a magnifying device such as a microscope or FACS analyser can be utilized as detection means. Beads are used for a simplified application because the production and processing of chips or other alternative carrier materials requires greater machinery. For "point-of-care diagnostics", the analysis of the beads can be carried out at the location using manageable available tools. These include, but are not limited to, suitable lenses, illumination systems and filters that allow the detection of bound viral particles using the camera of a standard smartphone or with the naked eye. In the case of a camera-based recording, the interpretation of the recorded image can be automated.

The simplicity of the method according to the invention makes it possible to carry out a large number of tests in a cost-effective and time-efficient manner. If the beads are rinsed with suitable rinsing agents, the beads can also be used repeatedly. It is preferred to elute the viruses from the beads without denaturing the binding agent bound to it in order to further reduce the associated material costs. The method according to the invention allows a local analysis of entire groups of people, for example the employees of a company, the passengers of a means of transport (e.g. aircraft) or the children and adolescents in day-care centres and schools. In the simplest case, the physical or biological means of detection is performed by the naked human eye, by which, for instance, a colour change can be detected.

A further aim of the method according to the invention is the enrichment of coronaviruses from a large sample volume. The sample is provided by or taken from a test person. Preferably, the sample is a body secretion such as saliva, blood serum, whole blood, sputum, urine, tear fluid or faeces. The method according to the invention also allows the analysis of samples taken via a rinse or swab (for example from the mouth, nose, ear, throat, intestine, urethra, vagina). For the detection of SARS-CoV-2 viruses that replicate in the throat, the sample to be analysed is preferably obtained by gargling an aqueous solution. Methods for enrichment are not claimed as such, only within the context of the method of detection as recited in claim 1.

The method according to the invention also enables pooled tests of entire groups of people to be carried out, for example, to detect people who are partially asymptomatic but are nevertheless in an infectious phase of a viral disease. The reagents used can be produced in large quantities at short notice and easily distributed. Since the detection of the coronavirus is not performed by amplifying parts of the viral genome, the method of the present invention cannot lead to a shortage of reagents, as has been observed, for example, with PCR-based tests. The actual performance of the test according to the invention is carried out on equipment that is widely available and accessible worldwide. The binding of coronaviruses to surfaces of the beads enables the enrichment even from large dilutions, so that pooled tests or tests of large sample volumes with small amounts of virus (such as waste water) are possible without fearing a significant loss of sensitivity. The sensitivity can be influenced by the type of subsequent detection and, in the case of a microscopic detection, by the choice of the staining agent. Preferably, staining agents are used which stain the RNA of the coronavirus. Preferred staining agents to be utilized in the present invention include, but are not limited to, SYBR, SYTOX, acridine orange (3-N,3-N,6-N,6-N-tetramethylacridine-3,6-diamine), thiazole orange, DAPI (4',6-diamidino-2-phenylindole), 7-AAD (7-aminoactinomycin D), ethidium bromide, propidium iodide. Alternatively, membrane staining agents can be used for staining the viral membranes, or staining agents that are coupled to secondary binding agents, for example marker- or conjugate-coupled antibodies. A preferred embodiment of the invention therefore comprises a colorimetric evaluation, preferably by utilizing an enzymatic or fluorescence-coupled staining agent.

For the detection of immobilised virus particles on the beads, quantitative analyses are not usually required, as it only matters to determine whether or not a patient contains virus particles in a sample that was taken from them. In this way, it is possible to identify infectious persons and prevent infections by isolating them at an early stage of infection. Applied on a large scale, this measure prevents a spread of coronaviruses that could trigger an epidemic or pandemic. If the amount of virus particles that is bound to the beads shall be quantitated, this can be done, for example, by means of a quantitative PCR or FACS analysis. However, the laboratory effort involved in such analyses is usually much higher, as the appropriate equipment and specialised personnel must be provided in order to perform quantitative studies. A microscopic quantification of the virus particles bound to the beads is also possible. Bound virus particles can either be counted individually or quantified based on the intensity of the overall signal. This can be done automatically using a suitable image analysis software.

Preferably the beads are individually labelled with an oligonucleotide that is specific for said carrier material. In a preferred embodiment, the carrier material is labelled, for example, in order to be able to assign samples to specific test persons. This can be done, for example, by coupling oligonucleotides with specific nucleotide sequences, or by using barcode technology. By such an implementation, the carrier material becomes addressable and thus can be used to identify specific binding events that occur on its surface. The use of specific marker sequences for such an identification is preferred.

The binding agent that is utilized in the present invention is selected from the group consisting of an angiotensin-converting enzyme 2 (ACE2), an ACE2 construct, an ACE2 fusion protein, or a modified or mutant ACE2 polypeptide or fusion protein that specifically recognizes and binds to a SARS-CoV-2 virus component. An alternative binding agent that is not used in the claimed invention can be an antibody or antibody fragment directed against viral components such as the spike S protein, including spike S1 protein, spike S2 protein, envelope protein, membrane protein, S-glycoprotein. Preferred antibodies are CR3022 antibody, CR3022-RB antibody, anti-spike antibodies, anti-spike S1 antibodies, anti-spike S2 antibodies, anti-envelope antibodies, anti-M antibodies, anti-S-glycoprotein antibodies or antibodies from sera of individuals who have survived infection. Included in the invention are all polyclonal, monoclonal antibodies or nanobodies suitable as binding agents and leading to the coupling of the virus, in particular the SARS-CoV-2 virus, to the carrier material. In particular, these include, but are not limited to, single chain antibodies, nanobodies and antibody mimetics such as, for example, adhirons, affibodies, affifins, affilins, anticalins, avimers, Armadillo repeat proteins, DARPins, fynomers, Kunitz domains, monomers and peptide aptamers. Such mimetics are created, for example, by an in vitro selection or in silico prediction, and structures of fibronectin III form the backbone of monobodies. Nanoparticles are particularly suitable for high-resolution light microscopic images and thus as detection means for staining the virus particles.

The binding agent for SARS-CoV-2, is angiotensin-converting enzyme 2 (ACE2), as well as constructs, multimers, hybrids, derivatives or fusion proteins derived therefrom. Such constructs may comprise full-length ACE2 or fragments thereof. A preferred variant for coupling to the carrier material is a modified, mutated or native ACE2 polypeptide or ACE2 fusion protein, preferably an Fc-ACE2 fusion protein or other tagged ACE2 fusion protein that specifically binds to the beads. For example, the ACE2 construct may be a modified ACE2 fusion protein, such as biotinylated Fc-ACE2, or multimers thereof. Preferably, the ACE2 protein that is utilized in the present invention as binding agent is human ACE2, which is coupled to the beads via suitable means, including, but not limited to biotin-streptavidin bindings.

In a preferred embodiment, a multimerisation of the binding molecules is provided. Each surface protein of a SARS-CoV-2 coronavirus according to the invention is present in a plurality of copies and can lead to a targeted multimerisation of the binding partners and consequently to a significantly stronger binding of virus particles. This is achieved by a direct binding to molecules immobilised in close proximity to each other on the beads, and alternatively or additionally by artificial multimerisation of these molecules. At the best, dimerisation takes place, e.g. via the Fc part of the binding molecule. More complex multimers are also conceivable, which, for example, are caused by dendritic structures. With a more precise knowledge of the viral surface, DNA origami can also be specifically applied in order to position the binding agent in a targeted and virus-specific manner. DNA origami involves the folding of DNA to create 2D and 3D objects at a nanoscale.

A combination of different binding agents is also possible. The combination of several binding agents leads to stronger binding of the RNA virus particles via avidity effects. In addition, a direct or indirect binding to beads, such as microparticles, of different sizes is possible.

The methods and kits of the present invention are also suitable for a multiplex application or a multiplex rapid test. Alternatively, or additionally, in a preferred embodiment, fluorescent carrier materials can be used, for example with fluorescent staining agent-labelled beads. Multiplexing can be easily realised via surfaces such as glass or plastic, if the sample is applied to different positions on the surface and subsequently tested for the SARS_CoV-2 virus.

Detection of stained virus particles takes place via physical, chemical or biological detection means, which include, but are not limited to, immunological or biotechnological detection means. An example of a physical detection means is the provision of an optical lens system for the optical detection of stained virus particles that are coupled to the carrier material. This can be done, for example, via a microscope, a FACS analyser, a microfluidic platform or the camera of a smartphone. If a microscopic detection is carried out, a non-specific visualisation of bound virus particles is possible.

RNA staining agents are used to detect immobilised coronavirus particles. Preferred RNA staining agents include, but are not limited, to SYTOX, SYBR, especially SYBR-Gold, acridine orange (3-N,3-N,6-N,6-N-tetramethylacridine-3,6-diamine), thiazole orange, DAPI (4',6-diamidino-2-phenylindole), 7-AAD (7-aminoactinomycin D), ethidium bromide, propidium iodide. Preferred membrane staining agents are lipophilic membrane staining agents such as Dil Stain (1,1'-dioctadecyl-3,3,3',3'-tetramethylindocarbocyanine perchlorate), or carbocyanine staining agents such as DiOC5 and DiOC6.

In a preferred embodiment, several detection methods can also be combined, resulting in a stronger signal response, for example a stronger fluorescence signal when fluorescent staining agents are used. Alternatively, the specificity of the detection can be increased by separate measurement and subsequent analysis of the spatial colocalisation.

As shown in the present invention, a microscopic detection can increase the specificity of the detection.

Moreover, a quantitative analysis allows the genome to be extracted from the virus particles immobilised on the beads and detected, for example, via sequencing, multiplex and real-time PCR or loop-mediated isothermal amplification (LAMP). The pre-binding and associated enrichment of the viruses increases the specificity and sensitivity of subsequent detection methods, which is due to the enrichment from a high sample volume such as environmental samples (e.g. consisting of waste water). The strong specific binding of the pathogens makes it possible due to the nature of the sample to be analysed by carrying out washing steps to eliminate impurities that are always present. This replaces time-consuming purification steps and facilitates subsequent detection methods, such as quantitative PCR, which requires high purity of the material to be analysed.

A native elution of specifically enriched SARS-CoV-2 viruses and the subsequent analysis of the SARS-CoV-2 viruses or their components preferably is effected via electron microscopy, fluorescence microscopy, mass spectrometry, proteomics, sequencing, PCR, ELISA, Western blotting, SDS PAGE, or chromatography. The viruses enriched in this way can themselves become the starting point for new methods, for example for the detection of antibodies in patients' sera or for the enrichment of pathogens in the production of vaccines. The SARS-CoV-2 virus particles enriched via specific binding agents can also bind antibodies from test persons without elution, similar to an ELISA. The specific detection of bound antibodies of a test person enables the detection of potentially neutralising antibodies in the tested sample. The detection of the bound antibody type (e.g. IgM, IgA or IgG) allows further conclusions to be drawn about the time and type of the infection process. Since the method according to the invention allows for specific enrichment of virus particles in high purity for subsequent analyses, a mild elution of bound virus particles by introducing a protease interface (e.g. TEV protease, SUMO protease) is possible. Alternative methods include Strep-Tag/Strep-Tactin^{™} with a subsequent elution with biotin, suitable tags such as ALFA-Tag (Nano-Tag), FLAG-Tag and subsequent elution by the free peptide. The utilisation of a poly-His-Tag and a subsequent elution with imidazole is also conceivable.

The primary objective of the invention is to provide a simple, robust, rapid and at the same time inexpensive detection method for SARS-CoV-2 viruses in the saliva of test persons in order to identify infectious persons and to prevent a further spread of the COVID-19 disease. The below examples illustrate the binding of coronaviruses via ACE2 and the subsequent visualisation of bound viruses via antibodies for the highly specific detection of SARS-CoV-2. According to the invention, the methods and kits of the invention are used for the detection of SARS-CoV-2, preferably using Fc-ACE2 fusion protein (or mutants or fragments thereof), as binding agent. The inventive method has proven to be surprisingly effective in detecting SARS-CoV-2, allowing simultaneous screening of many test persons with high throughput in a rapid and simple manner.

For the detection of SARS-CoV-2, streptavidin agarose or streptavidin-PMMA beads were used as preferred beads. In a preferred embodiment, SYBR-Gold is used as a staining agent for staining viral RNA. According to the invention, the cell surface receptor ACE2 was chosen as the primary target for coupling, as it is an essential receptor for the replication of the viruses in the target cells. Therefore, the virus will not be able to escape the detection by mutations. When human antibodies are used, it is likely that a selection pressure will occur which will limit their specificity, as the virus is going to escape detection by the human immune system. Binding of the virus to ACE2 will not be affected, but possibly other epitopes will be less efficient.

In a preferred embodiment of the invention, a fragment (Gln 18 - Ser 740) of human ACE2 that interacts with SARS-CoV-2 was used for the enrichment and detection of virus particles. Differently designed fragments of the protein, for example those containing the first 17 amino acids and binding the virus, can also possibly be used in preferred embodiments of the invention. Variants of ACE2 (e.g. protein fragments) or mutants (e.g. ACE2 with point mutations) that are able to bind the virus can be used in preferred versions of the invention, particularly if they show advantageous properties such as a higher binding capacity for binding to SARS-CoV-2, higher stability or ease of production. Possible variants also include ACE2 proteins from other organisms or species, such as the ACE2 isolated from bats, swine ACE2 or bovine ACE2.

It follows that a test according to the present invention also reflects the infectivity of the coronaviruses, because if a factor should prevent the binding of viruses to ACE2, for example due to the presence of IgA antibodies, it can be assumed that the secreted viruses have a significantly lower infectivity. Negative controls that can be used for the evaluation are beads without coupled binding agent and, or beads to which a binding agent is coupled that is not specific for the virus to be detected or isolated. Alternatively, point mutations in human ACE2 can be specifically introduced to prevent binding of SARS-CoV-2 to ACE2. The use of naturally occurring ACE2 sequences from other organisms that cannot be infected, e.g. murine ACE2, is also possible as controls in a test scenario that relates to the detection or enrichment of human coronaviruses.

The invention also relates to a kit for the detection of SARS-CoV-2 RNA viruses of the family *Coronaviridae,* comprising a binding agent selected from the group consisting of an angiotensin-converting enzyme 2 (ACE2), an ACE2 construct, an ACE2 fusion protein, or a modified or mutant ACE2 polypeptide or fusion protein that is capable of binding to a virus component of said SARS-CoV-2 RNA virus of the family *Coronaviridae,* beads that are coupled to the binding agent, a staining agent for viral RNA for staining viruses immobilised on the beads.

Preferably, a magnifying device is provided for making the staining agent visible to the human eye. Said magnifying device may also be part of a system for detecting RNA viruses comprising the features of the kit according to the invention. For a proper identification, the beads are preferably labelled with marker sequences, in particular oligonucleotides that bear a specific nucleotide sequence.

The beads in the kit usually consist of glass, polystyrene, PMMA or copolymers. In addition, membrane fragments, membrane vesicles in the form of colloids or combinations thereof are also conceivable. The particles may be coated and/or magnetic and/or electrically conductive and/or semiconductive. The beads, microspheres or microparticles used according to the invention can consist of different materials and are preferably particles of approximately the same size.

The methods and kits of the present invention create the conditions for quickly detecting infectious persons, which in particular is essential for preventing a coronavirus-related spread. This is a prerequisite to be able to dispense drastic measures such as lock-downs, and thus to enable a society to return to a largely normal everyday life despite the persistence of the pandemic. Tests can be carried out on a large scale, for example in schools or day-care centres, as diseases as COVID-19 are often asymptomatic in the groups of people represented there.

The method for the enrichment of coronaviruses (within the context of the detection method of claim 1) of the family *Coronaviridae* comprises the following steps: coupling a binding agent that is capable of binding to a virus component to a carrier material,
- incubating the carrier material with the thereon coupled binding agent with a virus-containing sample.

A preferred method for the enrichment of SARS-CoV-2 RNA viruses of the family *Coronaviridae* comprises the following steps:
- coupling a binding agent selected from the group consisting of an angiotensin-converting enzyme 2 (ACE2), an ACE2 construct, an ACE2 fusion protein, or a modified or mutant ACE2 polypeptide or fusion protein
   to a bead,
- incubating the beads with the thereon coupled binding agent with a SARS-CoV-2 virus-containing sample.

The virus is to be detected or enriched is SARS-CoV-2 and the binding agent is ACE2, preferably human ACE2, or a modified, truncated or mutant form thereof. In a further preferred embodiment, the binding agent is recombinant biotinylated Fc-ACE2 and/or the beads comprises streptavidin-agarose beads and/or the staining agent is SYBR Gold.

The method can be used, for example, to analyse samples of waste water by first enriching the virus particles contained therein according to the invention and then analysing said virus particles.

The methods and kits according to the invention are particularly suitable for the rapid and efficient detection of SARS-CoV-2 RNA viruses of the *Coronaviridae* family of both animal and human origin. It also includes SARS-CoV-2 RNA viruses of the *Coronaviridae* family that occur in animals such as mammalian or avian SARS-CoV-2 coronaviruses.

With the methods and kits according to the invention, it is possible to perform rapid high-throughput tests in a large population. At the same time, the enrichment procedure makes it possible to enrich viral samples, e.g. from a throat swab of a patient, for use in a subsequent PCR.

It is apparent that the invention also comprises any combinations of embodiments or features thereof.

### BRIEF DESCRIPTION OF THE DRAWINGS

Fig. 1 shows SARS-CoV-2 virus particles bound to biotinylated Fc-ACE2 coupled to streptavidin PMMA beads. The virus particles are visualised by SYBR Gold staining.
Fig. 2 shows SARS-CoV-2 bound beads that are stained with a combination of different binding agents anti-spike [1A9], anti-nucleoprotein, convalescent serum and the staining agent SYBR Gold.
Fig. 3 shows secondary methods for the detection of coronavirus in a sample using quantitative PCR, flow cytometry or colorimetry.
Fig. 4 shows SARS-CoV-2 detection from a patient swab using ACE2 beads.

### MODES FOR CARRYING OUT THE INVENTION

The invention is explained in more detail in the following embodiments.

### Binding of SARS-CoV-2 particles to PMMA microparticles

40 µl streptavidin-PMMA beads were incubated with 6 µg biotinylated Fc-ACE2 in a total volume of 100 µl PBS. To remove unbound proteins, beads were washed with PBS in Mobicol mini-columns with 10 µm filters inserted. The beads were resuspended in 0.5 ml PBS with 0.1% BSA.

0.5 ml cell culture supernatant containing 10⁶ SARS-CoV-2 particles was thawed and added to 12.5 µl PMMA beads (containing approximately 2000 beads) with pre-bound Fc-ACE2. Another source of SARS-CoV-2 particles resulted from a patient swab that was immersed in aqueous solution. The suspension consisting of virus particles and Fc-ACE2-coated beads was incubated for 2 hours at room temperature with constant gentle agitation. Beads with bound virus particles were collected and washed with PBS containing 0.1% BSA in Mobicol mini-columns with 10 µm filters inserted. Beads were resuspended in 250 µl PBS containing 0.1% BSA and fixed to inactivate virus by adding 250 µl 4% PFA (dissolved in PBS), followed by 30 min incubation at room temperature. The beads were washed with PBS containing 0.1% BSA and with PBS containing 50 mM Tris/HCl pH 7.5.

### Binding of SARS-CoV-2 particles to magnetic beads and subsequent analysis

To coat magnetic beads with Fc-ACE2, 2 ml magnetic Streptavidin-Dynabeads (4.5 µm diameter) at a concentration of 10⁷ beads / ml in PBS were incubated with 100 µg biotinylated Fc-ACE2. To remove unbound protein, magnetic beads were washed with PBS. Beads were resuspended in 0.5 ml PBS with 0.1% BSA and were distributed to 0.2 ml PCR tubes in either low or high amounts. The tubes containing low amount of magnetic beads contained only 1000 beads / tube and were used for experiments that aim at fluorescent detection of beads via flow cytometry. The tubes containing high amount of magnetic beads contained 750000 beads / tube and were used for experiments that aim at either qPCR analysis or detection via an enzyme-coupled colorimetric assay. In order to bind SARS-CoV-2 virus particles to magnetic beads 0.2 ml of either undiluted or the indicated ten-fold dilutions of the previously described cell culture supernatant was added to the Fc-ACE2-coated magnetic beads. The suspension was incubated for 2 hours at room temperature with constant gentle agitation. Beads with bound virus particles were collected, washed with PBS containing 0.1% BSA and PBS. Beads were either analysed by standard qPCR or fixed in 4% PFA (dissolved in PBS), followed by 30 min incubation at room temperature. The latter samples were washed with PBS containing 0.1% BSA and with PBS containing 50 mM Tris/HCl pH 7.5. Immunolabeling was performed as described below, followed either by standard FACS analysis or by an enzyme-based colorimetric assay as described below.

### Visualisation of SARS-CoV-2 particles via staining of the viral genome

Bound virus particles were stained with SYBR Gold at a final dilution of 1:50,000 in a final volume of 200 µl PBS. 50 µl of the bead suspension was transferred to a well of a µ-slide and images of the settled beads were taken with a confocal laser scanning microscope (Leica TCS SP5).

### Visualisation of SARS-CoV-2 particles via immunolabeling

To block unspecific interaction beads with bound virus particles were incubated for 30' in blocking buffer (PBS containing 0.1% (w/v) BSA and 0.1% (w/v) TX-100) at room temperature.

Labelling with mouse monoclonal antibodies was performed by a two-hour incubation with the primary antibody diluted in blocking buffer (mouse monoclonal SARS-CoV-2 spike antibody [1A9] applied at a final concentration of 2 µg/ml or mouse anti-nucleocapsid antibody (MM05) applied at a final concentration of 4 µg/ml) at room temperature. After two short washing steps (2x 250 µl blocking buffer, 10' each), the beads were incubated with secondary antibody (Alexa488-labelled anti-mouse IgG (H+L), obtained in goat, at a final concentration of 5 µg/ml in blocking buffer). After two short washing steps (2x 250 µl blocking buffer, 10' each), the bead suspension was transferred to a well of a µ-slide and images of the settled beads were taken with a confocal laser scanning microscope (Leica TCS SP5).

Labelling with convalescent serum was performed by a two-hour incubation with convalescent serum applied at a 1:800 dilution in blocking buffer at room temperature. After two short washing steps (2x 250 µl blocking buffer, 10' each), the beads were incubated with a mouse monoclonal anti-Ig kappa chain antibody (clone L1C1) at a final concentration of 5 µg/ml in blocking buffer. After two short washing steps (2x 250 µl blocking buffer, 10' each), the beads were incubated with secondary antibody (Alexa488-labelled anti-mouse IgG (H+L), obtained in goat, at a final concentration of 5 µg/ml in blocking buffer). After two short washing steps (2x 250 µl blocking buffer, 10' each), the bead suspension was transferred to a well of a µ-slide and images of the settled beads were taken with a confocal laser scanning microscope (Leica TCS SP5).

### Detection of SARS-CoV-2 virus particles bound to beads via an enzyme-coupled colorimetric assay

To block unspecific interaction beads with bound virus particles were incubated for 30' in blocking buffer (PBS containing 0.1% (w/v) BSA and 0.1% (w/v) TX-100) at room temperature. This was followed by a two-hour incubation with the primary mouse anti-nucleocapsid antibody (MM05) applied at a final concentration of 4 µg/ml in blocking buffer at room temperature. After two short washing steps (2x 250 µl blocking buffer, 10' each), the beads were incubated with secondary goat anti-mouse IgG (H+L) conjugated with horse radish peroxidase (HRP), at a final concentration of 1:500 in blocking buffer at room temperature. After extensive washing steps (8x 200 µl blocking buffer, 10' each), the bead suspension was transferred into new tubes and washed with 2x 200 µl PBS. Finally, beads were resuspended in 80 µl 1-Step Ultra TMB-ELISA Substrate Solution and incubated at room temperature. As soon as the colorimetric reaction reached an endpoint, aliquots were transferred into new tubes. Equal volumes of 2M sulfuric acid (H₂SO₄) were added which stopped the enzymatic reaction and resulted in a colour change to yellow.

### Materials

Biotinylated human Fc-ACE2: ACE2 (Gln 18 - Ser 740, Q9BYF1-1) - Fc (Pro 100 - Lys 330, P01857) - Avi; ACROBiosystems (Cat. AC2-H8F9)
Streptavidin PMMA beads, 21 µm functionalised monodisperse PMMA microparticles (PolyAn Cat. 105 21 020)
Magnetic Streptavidin-Dynabeads (4.5 µm diameter) = Invitrogen Exosome-Streptavidin Isolation/Detection Reagent; ThermoFisherScientific (Cat. 10608D)
Mobicol mini columns with inserted small 10 µm filter and Luer-Lock cap; MoBiTec (Cat. M105010S and Cat. M3009)
SYBR Gold nucleic acid stain, concentrate in DMSO; ThermoScientific (Cat. S11494)
SARS-CoV / SARS-CoV-2 (COVID-19) spike antibody [1A9]; Biozol (Cat. GTX-GTX632604); manufacturer: Genetex; host: mouse; clone 1A9, isotype IgG1
SARS-CoV/SARS-CoV-2 Nucleocapsid Antibody, Mouse MAb; Hölzel Diagnostika Handels GmbH (Cat. 40143-MM05-100)
Mouse monoclonal Anti-Ig kappa chain antibody clone L1C1, raised against B lymphoma cells of human origins, SantaCruz Biotechnologies (Cat. sc-59265)
Goat anti-Mouse IgG (H+L) Highly Cross-Adsorbed Secondary Antibody, Alexa Fluor 488; ThermoScientific (Cat. #A-11029)
Goat anti-Mouse IgG (H+L) Highly Cross-Adsorbed Secondary Antibody, HRP; ThermoScientific (Cat. #A16078)
1-Step Ultra TMB-ELISA Substrate Solution, ThermoScientific (Cat. #34028)
µ-slide angiogenesis glass bottom; Ibidi cat. 81507

### Detailed description of the Figures:

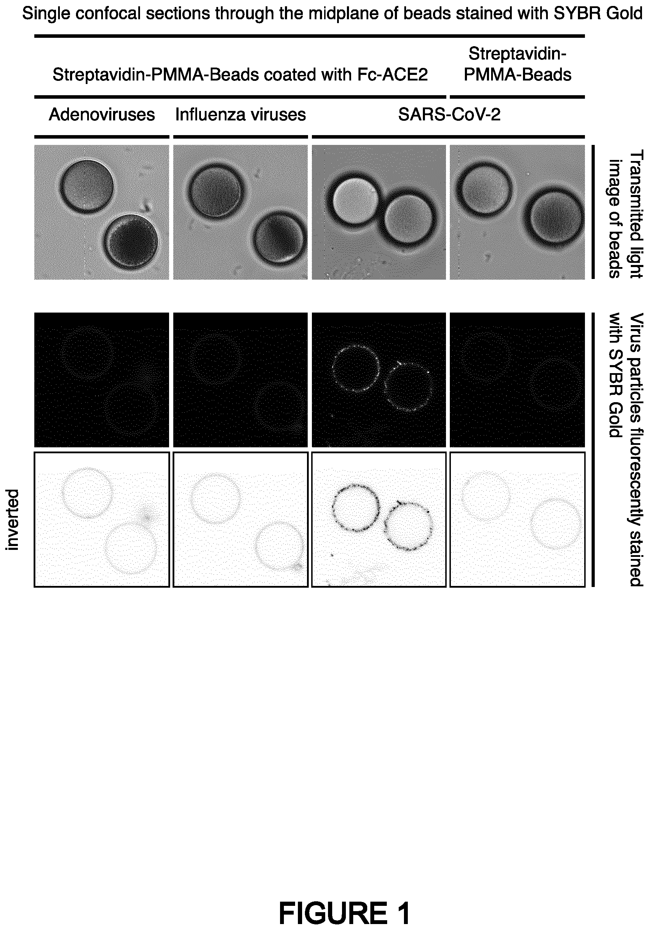

**Figure 1** shows the specific enrichment of SARS-CoV-2 virus particles on the surface of Fc-ACE2 coated beads and instantaneous staining of bound virus particles with a nucleic acid dye as an experimental method and diagnostic tool.

The specific interaction between human ACE2 and the spikes of SARS-CoV-2 mediates viral binding to human cells and their subsequent infection. To test whether this interaction can be used for the enrichment of intact viruses on carrier materials, streptavidin-PMMA-Beads were coated with biotinylated Fc-ACE2. The specificity of such Fc-ACE2-coated beads was evaluated by incubating them with cell culture supernatants containing the indicated virus particles (adenovirus, influenza virus or SARS-CoV-2). Streptavidin-PMMA-Beads served as additional control for specificity. After.incubation beads were washed to remove unbound material and fixed with PFA. Bound virus particles were fluorescently stained with the nucleic acid dye SYBR Gold. Fluorescence microscopic analysis was performed by confocal laser scanning microscopy and single confocal sections recorded with identical microscopy settings are shown. The transmitted light images serve to locate the beads. The beads shown in Figure 1 are highly representative for other beads processed and analyzed under identical experimental conditions.

Binding of SARS-CoV-2 was not detected on empty beads (streptavidin-PMMA-beads) that served as negative control. Thus, SARS-CoV-2 enrichment on Fc-ACE2-coated beads was dependent on the presence of its specific binder, Fc-ACE2. While SARS-CoV-2 binding was very efficient yielding characteristic and bright fluorescent dots neither influenza virus nor adenovirus particles were detected on beads coated with Fc-ACE2. In conclusion, beads coated with Fc-ACE2 allowed for the highly specific enrichment of SARS-CoV-2 and can therefore serve as a diagnostic tool to detect the presence of intact and therefore potentially infectious virus particles. As shown here, an easy and rapid way to detect bound virus particles is to perform subsequent staining of viral genome with a nucleic acid dye. Due to electrostatic interactions with the bead surface SYBR Gold displays a very dim and unspecific background staining. As shown in Figure 1, such background staining can be easily discerned from the characteristic punctate and very bright pattern of stained virus particles. The surface of the beads used as well as the buffer conditions can be further optimized to suppress unspecific staining and thereby strongly increase the signal-to-noise ratio.

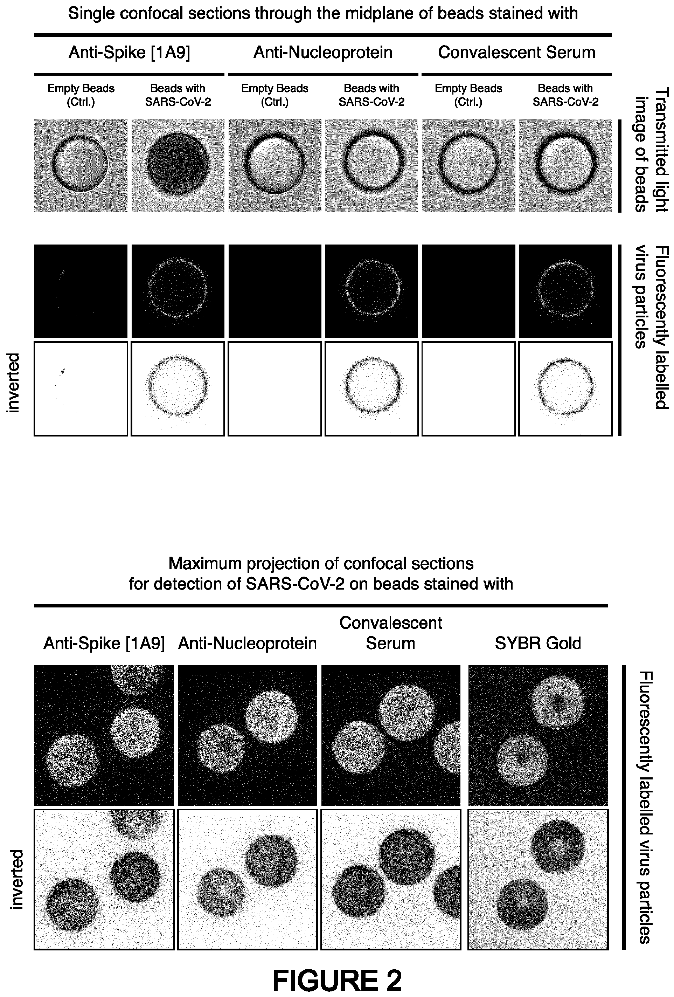

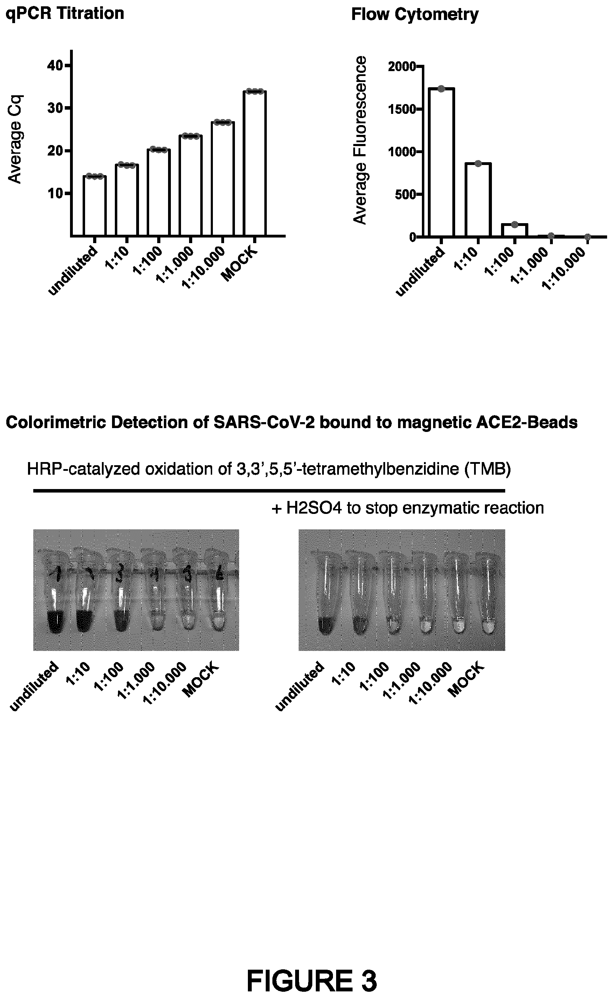

In summary, Figure 1 shows that SARS-CoV-2 virus particles could be specifically enriched on carrier materials like beads coated with a binding agent (here ACE2) that specifically bind to viral surface proteins. Bound virus particles were readily detected by instantaneous staining of viral genomes using SYBR Gold. Although images shown here have been recorded with confocal laser scanning microscope, analysis can be performed using more simple and standard fluorescent microscopes or even mobile, handheld devices to allow rapid point-of-care diagnostic. Fc-ACE2-coated beads thus allow the selective enrichment of SARS-CoV-2 viruses and form the basis for sensitive diagnostics, e.g. via staining of the viral genome using SYBR Gold (Figure 1), via immunolabeling of the viruses (Figure 2, 3 and 4) or via nucleic acid-based diagnostics such as established PCR-based detection methods (Figure 3).

**Figure 2** shows methods of immunolabelling of SARS-CoV-2 particles which have been specifically enriched on beads coated with Fc-ACE2.

In addition to staining the viral genome, immunolabeling of bound viruses presents an alternative method for detecting virus particles bound to a carrier material. The use of antibodies is expected to increase both the specificity and sensitivity of the virus detection. The feasibility of such a method was tested on beads on which viruses had been enriched.

The experiment shown in the upper panel makes use of streptavidin-PMMA-Beads beads or streptavidin-PMMA-Beads coated with recombinant biotinylated Fc-ACE2. Both types of beads were incubated with a cell culture supernatant containing SARS-CoV-2. After washing away unbound material beads were fixed with PFA. The described procedure resulted in two types of beads, streptavidin-PMMA-Beads without SARS-CoV-2 particles (Empty Beads) and Fc-ACE2 coated beads with bound SARS-CoV-2 (Beads with SARS-CoV-2). Bound SARS-CoV-2 particles were immunolabelled with commercially available monoclonal antibodies recognizing either the spike protein or the nucleocapsid protein of SARS-CoV-2. A third source of antibodies (polyclonal human antibodies) for immunolabelling was serum obtained from a patient who recovered from a previous infection with SARS-CoV-2 (convalescent serum). In the course of immunofluorescence, the beads covered with SARS-Cov-2 were incubated with the indicated antibodies against SARS-CoV-2 proteins. Fluorescently labelled polyclonal antibodies were used as secondary antibodies. Stained virus particles were visualized by confocal laser scanning microscopy and single confocal sections are shown. Importantly, the beads that were covered with SARS-CoV-2 particles were recorded with the same microscopy settings as the corresponding empty beads, which served as the negative controls. As beads lacking SARS-CoV-2 virus particles did not result in any detectable fluorescent staining, the various ways to immunolabel SARS-CoV-2 particles were considered highly specific.

The experiment demonstrates that readily available proteinaceous binders (here antibodies) against viral proteins can be used to specifically detect bound virus particles. Although resulting in an even brighter and more specific staining, the procedure is inherently not as rapid as a nucleic acid stain shown in Figure 1. However, experimental protocols can be optimized for fast staining that can be completed in the range of minutes. Similarly, sensitivity of such assay can be improved further, for example by introducing additional fluorescent staining agents or combining different staining methods.

The lower panel shows maximum projections of recorded stacks of confocal sections (different planes in z). This visualization of microscopy data unveils the characteristic dot-like staining pattern of bound virus particles. The various methods used to detect bound virus particles yielded very similar results, demonstrating the robustness of workflows based on the fluorescent detection of virus particles.

**Figure 3** shows alternative methods of detection of SARS-CoV-2 which has been enriched on beads coated with Fc-ACE2.

To test alternative methods of labelling and detection, streptavidin magnetic beads coated with recombinant biotinylated Fc-ACE2 were incubated with ten-fold serial dilutions of a cell culture supernatant containing SARS-CoV-2. After washing away unbound material beads were either directly analyzed by qPCR or fixed with PFA. The graph qPCR Titration illustrates that beads coated with Fc-ACE2 enrich SARS-CoV-2 virus particles that can subsequently be detected and quantified by nucleic acid based-diagnostics such as established PCR-based detection methods.

In order to perform flow cytometry analysis Fc-ACE2 coated magnetic streptavidin beads with bound SARS-CoV-2 were fixed and virus particles were specifically immunolabelled with the commercially available monoclonal antibodies recognizing the nucleocapsid protein of SARS-CoV-2. Fluorescently labelled polyclonal antibodies were used as secondary antibodies and beads with bound virus particles were analyzed by flow cytometry. The graph shown relates measured values of fluorescence to the amount of virus particles bound to beads. This experiment demonstrates that classical flow cytometry or related methods represent a highly suitable method for detecting and quantifying virus particles bound to beads.

The use of enzymatic activities such as that of horseradish peroxidase, luciferase or alkaline phosphatase allows a targeted amplification of the signal for the highly sensitive detection of viruses bound to the carrier material. To test the applicability of such method, bound virus particles were specifically immunolabelled with a monoclonal antibody recognizing the nucleocapsid protein of SARS-CoV-2. The secondary antibodies were polyclonal antibodies coupled to horse radish peroxidase. The actual method of detection is based on an enzymatic reaction mediated by horse-radish peroxidase. The colour change after addition of the substrate TMB is a highly sensitive diagnostic feature and its intensity depends on the amount of bound SARS-CoV-2 virus particles. Figure 3 shows that such colorimetric assay can be used for quantification either before or after stopping the reaction by addition of H₂SO₄.

In summary, Figure 3 demonstrates three additional methods to detect virus particles specifically bound to beads. Besides being sensitive and specific, the presented methods are highly suitable for quantifying virus particles bound to beads over a wide range of concentrations.

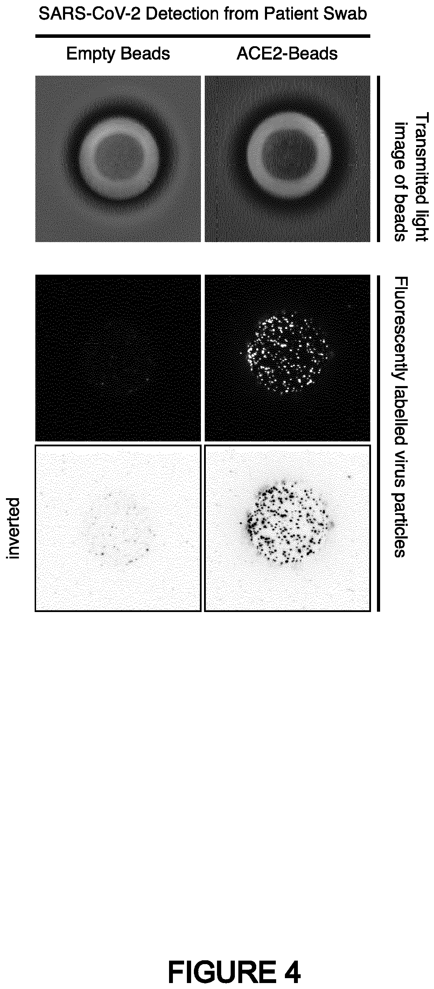

**Figure 4** shows the detection of SARS-CoV-2 virus in patient sample and demonstrates that a microscopic approach is able to reliably enrich and detect single virus particles even from patient samples.

A nasopharyngeal swab taken from a diagnosed COVID19 patient was immersed in buffer to dissolve virus particles in buffered aqueous solution. The supernatant containing solubilized virus particles was transferred either on streptavidin-PMMA-Beads (empty beads) or streptavidin-PMMA-Beads coated with biotinylated Fc-ACE2 (ACE2 beads). Unbound material was washed away, beads were fixed with PFA and processed like in Figure 2 by incubation with the monoclonal anti-nucleocapsid antibody followed by an incubation with fluorescently labelled secondary antibodies. Fluorescence was visualized by confocal laser scanning microscopy. Maximum projections of recorded stacks of confocal sections (different planes in z) are shown. Like in the lower panel of Figure 2 this type of visualization of microscopy data unveils the characteristic dot-like staining pattern of bound virus particles.

Since beads are used to enrich for viruses, sensitivity largely depends on the sample volume and the actual amount of beads used in the assay. As demonstrated here, specific enrichment combined with subsequent staining of bound virus particles can be used to unequivocally diagnose a real sample taken from a diagnosed patient.

## Claims

1. A method for the detection of SARS-CoV-2 RNA viruses of the family *Coronaviridae,* comprising
- coupling a binding agent selected from the group consisting of an angiotensin-converting enzyme 2 (ACE2), an ACE2 construct, an ACE2 fusion protein, or a modified or mutant ACE2 polypeptide or ACE2 fusion protein that is capable of binding to a virus component of said SARS-CoV-2 RNA virus of the family *Coronaviridae* to beads,
- incubating the beads with the thereon coupled binding agent with a SARS-CoV-2 virus-containing sample,
- staining the viruses immobilised on the beads with a staining agent for viral RNA,
- detecting stained virus particles via a physical, chemical or biological detection means.

2. The method according to claim 1, wherein the SARS-CoV-2 RNA virus of the *Coronaviridae* family is selected from the group consisting of feline, canine, porcine, bovine, bat, pangolin, ferret, mink, bird or dromedary camel SARS-CoV-2 coronavirus.

3. The method of any one of claims 1 or claim 2, wherein the staining agent for viral RNA is selected from the group consisting of SYTOX, SYBR, SYBR Gold, acridine orange (3-N,3-N,6-N,6-N-tetramethylacridine-3,6-diamine), thiazole orange, DAPI (4',6-diamidino-2-phenylindole), 7-AAD (7-aminoactinomycin D), ethidium bromide, propidium iodide, an enzymatic or fluorescence-coupled staining agent for viral RNA.

4. The method according to any one of claims 1 to 3, wherein the physical detection means is a FACS analyzer, a microfluidic platform, a microscope, a confocal laser scanning microscope, a camera and/or the human eye.

5. The method according to any one of claims 1 to 4, wherein the biological detection means is a conjugate- or marker-coupled further secondary binding agent.

6. The method according to any one of claims 1 to 5, wherein the sample is taken from body secretions such as saliva, blood serum, whole blood, sputum, urine, tear fluid, faeces, a rinse or swab (in particular from the mouth, nose and/or throat) or a gargle sample.

7. The method according to any one of claims 1 to 6, wherein the binding agent is recombinant biotinylated Fc-ACE2.

8. The method according to any one of claims 1 to 7 wherein the beads are streptavidin-agarose beads or streptavidine PMMA.

9. A kit for the detection of SARS-CoV-2 RNA viruses of the family *Coronaviridae,* comprising:
a. a binding agent selected from the group consisting of an angiotensin-converting enzyme 2 (ACE2), an ACE2 construct, an ACE2 fusion protein, or a modified or mutant ACE2 polypeptide or ACE2 fusion protein that specifically recognizes and binds to a virus component of said SARS-CoV-2 RNA virus of the family *Coronaviridae,*
b. beads that are coupled to the binding agent,
c. a staining agent for viral RNA for staining viruses immobilised on the beads.

10. The kit according to claim 9, wherein the SARS-CoV-2 RNA virus of the *Coronaviridae* family is selected from the group consisting of feline, canine, porcine, bovine, bird or dromedary camel SARS-CoV-2 coronavirus.

11. The kit according to any one of claims 9 or 10, wherein the staining agent for viral RNA is selected from the group consisting of SYTOX, SYBR, SYBR Gold, acridine orange (3-N,3-N,6-N,6-N-tetramethylacridine-3,6-diamine), thiazole orange, DAPI (4',6-diamidino-2-phenylindole), 7-AAD (7-aminoactinomycin D), ethidium bromide, propidium iodide, an enzymatic or fluorescence-coupled staining agent for viral RNA.

12. The kit according to any one of claims 9 to 11, wherein the beads are streptavidin-agarose beads or streptavidine PMMA.

13. The kit according to any one of claim 9 to 12, wherein a magnifying device is provided for making the staining agent for viral RNA visible to the human eye.

14. The kit according to any one of claims 9 to 13, wherein the beads are individually labelled with an oligonucleotide that is specific for said beads.

## Patentansprüche

1. Verfahren zum Nachweis von SARS-CoV-2-RNA-Viren der Familie *Coronaviridae,* umfassend
- Koppeln eines Bindeagens, ausgewählt aus der Gruppe bestehend aus einem Angiotensin-konvertierenden Enzym 2 (ACE2), einem ACE2-Konstrukt, einem ACE2-Fusionsprotein oder einem modifizierten oder mutierten ACE2-Polypeptid oder einem ACE2-Fusionsprotein, das in der Lage ist, an eine Viruskomponente des SARS-CoV-2-RNA-Virus aus der Familie *Coronaviridae* an Beads zu binden,
- Inkubieren der Beads mit dem daran gekoppelten Bindemittel mit einer SARS-CoV-2-virushaltigen Probe,
- Färben der auf den Beads immobilisierten Viren mit einem Färbemittel für virale RNA,
- Detektieren von gefärbten Viruspartikeln über ein physikalisches, chemisches oder biologisches Detektionsmittel.

2. Verfahren nach Anspruch 1, wobei das SARS-CoV-2-RNA-Virus der Familie *Coronaviridae* ausgewählt ist aus der Gruppe bestehend aus Katzen-, Hunde-, Schweine-, Rinder-, Fledermaus-, Schuppentier-, Frettchen-, Nerz-, Vogel- oder Dromedar-SARS-CoV-2-Coronavirus.

3. Verfahren nach einem der Ansprüche 1 oder 2, wobei das Färbemittel für virale RNA ausgewählt ist aus der Gruppe bestehend aus SYTOX, SYBR, SYBR Gold, Acridin-Orange (3-N,3-N,6-N,6-N-Tetramethylacridin-3,6-diamin), Thiazol-Orange, DAPI (4',6-Diamidino-2-phenylindol), 7-AAD (7-Aminoactinomycin D), Ethidiumbromid, Propidiumlodid, einem enzymatischen oder fluoreszenzgekoppelten Färbemittel für virale RNA.

4. Verfahren nach einem der Ansprüche 1 bis 3, wobei das physikalische Detektionsmittel ein FACS-Analysegerät, eine mikrofluidische Plattform, ein Mikroskop, ein konfokales Laser-Scanning-Mikroskop, eine Kamera und/oder das menschliche Auge ist.

5. Verfahren nach einem der Ansprüche 1 bis 4, wobei das biologische Detektionsmittel ein Konjugat- oder Marker-gekoppeltes weiteres sekundäres Bindeagens ist.

6. Verfahren nach einem der Ansprüche 1 bis 5, wobei die Probe aus Körpersekreten wie Speichel, Blutserum, Vollblut, Sputum, Urin, Tränenflüssigkeit, Fäkalien, einer Spülung oder einem Abstrich (insbesondere aus dem Mund, der Nase und/oder dem Rachen) oder einer Gurgelprobe entnommen wird.

7. Verfahren nach einem der Ansprüche 1 bis 6, wobei das Bindeagens rekombinantes biotinyliertes Fc-ACE2 ist.

8. Verfahren nach einem der Ansprüche 1 bis 7, wobei die Beads Streptavidin-Agarose-Beads oder Streptavidin-PMMA sind.

9. Kit zum Nachweis von SARS-CoV-2-RNA-Viren der Familie *Coronaviridae,* umfassend
a) ein Bindeagens, ausgewählt aus der Gruppe bestehend aus einem Angiotensin-konvertierenden Enzym 2 (ACE2), einem ACE2-Konstrukt, einem ACE2-Fusionsprotein oder einem modifizierten oder mutierten ACE2-Polypeptid oder einem ACE2-Fusionsprotein, das spezifisch eine Viruskomponente des SARS-CoV-2-RNA-Virus aus der Familie *Coronaviridae* erkennt und daran bindet,
b) Beads, die an das Bindeagens gekoppelt sind,
c) ein Färbemittel für virale RNA zum Färben der auf den Beads immobilisierten Viren.

10. Kit nach Anspruch 9, wobei das SARS-CoV-2-RNA-Virus der Familie *Coronaviridae* ausgewählt ist aus der Gruppe bestehend aus Katzen-, Hunde-, Schweine-, Rinder-, Vogel- oder Dromedar-SARS-CoV-2-Coronavirus.

11. Kit nach einem der Ansprüche 9 oder 10, wobei das Färbemittel für virale RNA ausgewählt ist aus der Gruppe bestehend aus SYTOX, SYBR, SYBR Gold, Acridin-Orange (3-N,3-N,6-N,6-N-Tetramethylacridin-3,6-diamin), Thiazol-Orange, DAPI (4',6-Diamidino-2-phenylindol), 7-AAD (7-Aminoactinomycin D), Ethidiumbromid, Propidiumlodid, einem enzymatischen oder fluoreszenzgekoppelten Färbemittel für virale RNA.

12. Kit nach einem der Ansprüche 9 bis 11, wobei die Beads Streptavidin-Agarose-Beads oder Streptavidin-PMMA sind.

13. Kit nach einem der Ansprüche 9 bis 12, wobei eine Vergrößerungseinrichtung vorgesehen ist, um das Färbemittel für virale RNA für das menschliche Auge sichtbar zu machen.

14. Kit nach einem der Ansprüche 9 bis 13, wobei die Beads einzeln mit einem Oligonukleotid markiert sind, das spezifisch für die Beads ist.

## Revendications

1. Procédé pour la détection des virus à ARN SARS-CoV-2 de la famille des *Coronaviridées,* comprenant :
- le couplage d'un agent de liaison qui est sélectionné parmi le groupe qui est constitué par une enzyme de conversion de l'angiotensine de type 2 (ACE2), une construction de l'ACE2, une protéine de fusion de l'ACE2 ou un polypeptide de l'ACE2 modifié ou mutant ou une protéine de fusion de l'ACE2 modifiée ou mutante qui dispose de la capacité de se lier à un composant viral dudit virus à ARN SARS-CoV-2 de la famille des *Coronaviridées* sur des billes ;
- l'incubation des billes tandis que l'agent de liaison leur est couplé avec un échantillon qui contient le virus à ARN SARS-CoV-2 ;
- la coloration des virus qui sont immobilisés sur les billes à l'aide d'un agent de coloration pour l'ARN viral ; et
- la détection de particules virales colorées via un moyen de détection physique, chimique ou biologique.

2. Procédé selon la revendication 1, dans lequel le virus à ARN SARS-CoV-2 de la famille des *Coronaviridées* est sélectionné parmi le groupe qui est constitué par le coronavirus SARS-CoV-2 félin, canin, porcin, bovin, de la chauve-souris, du pangolin, du furet, du vison, des oiseaux ou du dromadaire.

3. Procédé selon l'une quelconque de la revendication 1 ou de la revendication 2, dans lequel l'agent de coloration pour l'ARN viral est sélectionné parmi le groupe qui est constitué par le SYTOX, le SYBR, le SYBR Gold, l'orange d'acridine (3-N,3-N,6-N,6-N-tétraméthylacridine-3,6-diamine), le thiazole orange, le DAPI (4',6-diamidino-2-phénylindole), le 7-AAD (7-aminoactinomycine D), le bromure d'éthidium, l'iodure de propidium, un agent de coloration enzymatique ou couplé par fluorescence pour l'ARN viral.

4. Procédé selon l'une quelconque des revendications 1 à 3, dans lequel le moyen de détection physique est un analyseur FACS, une plateforme micro-fluidique, un microscope, un microscope laser à balayage confocal, une caméra et/ou l'œil de l'être humain.

5. Procédé selon l'une quelconque des revendications 1 à 4, dans lequel le moyen de détection biologique est un autre agent de liaison secondaire conjugué ou couplé à un marqueur.

6. Procédé selon l'une quelconque des revendications 1 à 5, dans lequel l'échantillon est prélevé à partir de sécrétions corporelles telles que la salive, le sérum du sang, le sang complet, les expectorations, l'urine, le liquide lacrymal, les matières fécales, un échantillon obtenu à partir d'un badigeonnage ou rinçage ou à partir d'un écouvillonnage ou prélèvement (en particulier, à partir de la bouche, du nez et/ou de la gorge) ou un échantillon obtenu à partir d'un gargarisme.

7. Procédé selon l'une quelconque des revendications 1 à 6, dans lequel l'agent de liaison est le Fc-ACE2 biotinylé recombinant.

8. Procédé selon l'une quelconque des revendications 1 à 7, dans lequel les billes sont des billes d'agarose à streptavidine ou sont à base de polyméthacrylate de méthyle/PMMA à streptavidine.

9. Kit pour la détection des virus à ARN SARS-CoV-2 de la famille des *Coronaviridées,* comprenant :
a. un agent de liaison qui est sélectionné parmi le groupe qui est constitué par une enzyme de conversion de l'angiotensine de type 2 (ACE2), une construction de l'ACE2, une protéine de fusion de l'ACE2 ou un polypeptide de l'ACE2 modifié ou mutant ou une protéine de fusion de l'ACE2 modifiée ou mutante qui reconnaît de façon spécifique un composant viral dudit virus à ARN SARS-CoV-2 de la famille des *Coronaviridées* et se lie de façon spécifique à celui-ci ;
b. des billes qui sont couplées à l'agent de liaison ; et
c. un agent de coloration pour l'ARN viral, pour colorer les virus qui sont immobilisés sur les billes.

10. Kit selon la revendication 9, dans lequel le virus à ARN SARS-CoV-2 de la famille des *Coronaviridées* est sélectionné parmi le groupe qui est constitué par le coronavirus SARS-CoV-2 félin, canin, porcin, bovin, de la chauve-souris, du pangolin, du furet, du vison, des oiseaux ou du dromadaire.

11. Kit selon l'une quelconque de la revendication 9 ou de la revendication 10, dans lequel l'agent de coloration pour l'ARN viral est sélectionné parmi le groupe qui est constitué par le SYTOX, le SYBR, le SYBR Gold, l'orange d'acridine (3-N,3-N,6-N,6-N-tétraméthylacridine-3,6-diamine), le thiazole orange, le DAPI (4',6-diamidino-2-phénylindole), le 7-AAD (7-aminoactinomycine D), le bromure d'éthidium, l'iodure de propidium, un agent de coloration enzymatique ou couplé par fluorescence pour l'ARN viral.

12. Kit selon l'une quelconque des revendications 9 à 11, dans lequel les billes sont des billes d'agarose à streptavidine ou sont à base de polyméthacrylate de méthyle/PMMA à streptavidine.

13. Kit selon l'une quelconque des revendications 9 à 12, dans lequel un dispositif d'agrandissement est prévu pour rendre visible à l'œil de l'être humain l'agent de coloration pour l'ARN viral.

14. Kit selon l'une quelconque des revendications 9 à 13, dans lequel les billes sont étiquetées de façon individuelle à l'aide d'un oligonucléotide qui est spécifique pour lesdites billes.
